Europäisches Patentamt

European Patent Office

Office européen des brevets

(19)

(11) Publication number: **0 011 865**
**B1**

(12) **EUROPEAN PATENT SPECIFICATION**

(45) Date of publication of patent specification: **19.05.82**

(51) Int. Cl.³: **A 61 K 39/12 //C12N7/00**

(21) Application number: **79104764.0**

(22) Date of filing: **29.11.79**

(54) Feline infectious peritonitis vaccine and process for its preparation.

(30) Priority: **30.11.78 GB 4666678**

(43) Date of publication of application:
**11.06.80 Bulletin 80/12**

(45) Publication of the grant of the patent:
**19.05.82 Bulletin 82/20**

(84) Designated Contracting States:
**AT BE CH DE FR GB IT NL SE**

(56) References cited:
GB - A - 1 177 635
GB - A - 1 286 250
GB - A - 1 471 262
GB - A - 1 492 930

(73) Proprietor: THE WELLCOME FOUNDATION
LIMITED
183-193 Euston Road
London NW1 2BP (GB)

(72) Inventor: Fishman, Bernard
7 Rowlands Close
Gillingham, Kent (GB)
Inventor: O'Reilly, Kevin Joseph
Lambardes Drakeley's Field
Milland Near Liphook, Hants (GB)
Inventor: Hitchcock, Louise Mary
65 Kingswood Avenue
Shortlands Bromley, Kent (GB)

(74) Representative: Berg, Wilhelm, Dr. et al,
Dr. Berg, Dipl.-Ing. Stapf, Dipl.-Ing. Schwabe, Dr.
Dr. Sandmair Mauerkircherstrasse 45
D-8000 München 80 (DE)

Courier Press, Leamington Spa, England.

# Feline infectious peritonitis vaccine and process for its preparation

This invention relates to a method of preparing an inactivated feline infectious peritonitis vaccine, hereinafter referred to as FIP vaccine, and in particular to a vaccine made by the method, which is useful in the prevention of FIP. In a further aspect the invention relates to a method for developing immunity to FIP in animals of the genus *Felix*.

Feline infectious peritonitis is an important disease of both domestic and wild *felidae* because it is almost invariably fatal. The disease was first recognised in about 1950 and reported under the name chronic fibrinous peritonitis (*J. Amer. Vet. Med. Assoc., 144,* 1409—1420 (1963)). Since then FIP has been diagnosed in North America, Europe, South Africa, Australasia and Japan. More recently the causative organism has been described as a corona-like virus (*Nature, 266,* 682 (1977)).

Animals which contract the disease become lethargic and inappetent. Fluid collects in the peritoneal cavity with the result that the abdomen becomes enlarged and the cat eventually dies in a jaundiced state. Whilst therapy and supportive treatment may prolong the course of the disease, all animals showing clinical signs eventually die (*J. Amer. Vet. Med. Assoc., 154,* 26—35 (1969)).

It has long been recognised that a vaccine is required to prevent FIP outbreaks (*J. Amer. Vet. Med. Assoc., 155,* 1728—1733 (1969)), however many attempts to isolate and grow the virus have failed. It has recently been reported that FIP virus has been grown in feline small intestine organ culture (*The Cornell Veterinarian, 68,* 411—417 (1978)), but this is not a practical method for vaccine production.

It has been found that an inactivated FIP vaccine can be prepared by passaging an isolate of virulent FIP in feline embryonic cell cultures until the infected cultures exhibit a characteristic cytopathic effect (CPE), as hereinafter described, inactivating the virus so produced, and presenting the virus in association with a pharmaceutically acceptable carrier.

According to the present invention in one aspect there is provided a method for preparing an inactivated feline infectious peritonitis vaccine comprising serially passaging an isolate of virulent FIP in feline embryonic cell cultures until the infected cultures exhibit a CPE characteristic of FIP, inactivating the virus so produced, and presenting the inactivated virus in association with a pharmaceutically acceptable carrier.

Virulent FIP virus may be isolated from the peritoneal fluid, blood, urine and various organs of a cat suffering from the disease. In the case of the peritoneal fluid, the urine, the infected fluid may be inoculated directly on to the selected cell cultures. For isolating virus from the blood the infected animal may be bled from the jugular or any other suitable vein. The sample is subsequently allowed to clot, is then removed and triturated, for example in a pestle and mortar, and resuspended in a suitable medium, such as phosphate buffered saline. The suspension is then centrifuged to remove gross tissue debris, the supernatant fluid is removed and used to inoculate cell cultures. Virus may be obtained from such organs as the liver, spleen or omentum; the procedure for preparing the virus for inoculation into cultures is essentially similar to that for the clotted blood, in that the organ is triturated, resuspended, followed by centrifugation and removal of the supernatant fluid for inoculation purposes.

Feline embryonic cell cultures suitable for serially passaging the virulent virus may be derived from such organs as lung, kidney, heart, liver or gut, or a mixture thereof, or alternatively from the skin, muscle, amnion (placenta) or tongue. Cell cultures may be prepared from the various parts of a feline embryo, or even the whole embryo, by the method described in British Patents Nos. 1 177 635 and 1 286 250 and also in *J. Hygiene, 67* (1969), 115—124. The former patent describes a method for preparing diploid cell strains, whilst the latter describes the preparation of heteroploid cell lines. The methods comprise taking the embryo, or the required part of it, and disaggregating it mechanically or enzymatically. The resulting cell suspension is transferred into suitable containers holding a nutrient medium suitable for culturing cells, and the incubating at 32° to 39°C. To facilitate subculturing it is customary to treat the confluent sheets of cells with trypsin and an innocuous chelating agent, before each transfer into fresh medium. Alternatively primary feline cell cultures may be used for growing FIP virus, and these are prepared by techniques well known in the art.

The isolated virulent FIP virus may be propagated by inoculating a confluent monolayer or sheet of the selected feline embryonic cells with the isolate from the infected animal, followed by incubation at 35°C to 39°C, preferably 37°C, for from 3 to 10 days, preferably 5 to 8 days, in the presence of a suitable medium. Desirably the medium is chemically defined with no biological additives, for example the medium is preferably serum-free. Such media as Eagle's Basal Medium (Eagle H., *Science* (1959), *130,* 432) or 199 Maintenance Medium (see Morgan, J. F. et al, *Proc. Soc. Exp. Biol. (N.Y.),* 73, 1 (1950)) are suitable. Antibacterial agents such as penicillin and/or streptomycin may be added to the medium. After incubation the virus can be harvested by mechanical means such as freezing and thawing, and thereafter used for serially passaging in monolayer cultures of feline embryonic cells by standard tissue culture techniques (see "A Manual of Basic Virological Techniques" by G. C. Rovozzo and C. N. Burke, published by Prentice-Hall Inc., Englewood Cliffs, N.J. U.S.A., 1973).

During the first few passages of the virus, usually up to 5 subcultures, no obvious cytopathic effect (CPE) is visible. Thereafter FIP virus infection of cell monolayers characteristically causes the infected cells to produce irregular shapes, when examined microscopically as wet preparations, and ultimately round up and/or become detached from the solid surface on which they are growing. Thus ultimately the virus vompletely destroys the monolayer culture.

Under the electron microscope FIP virus has an appearance similar to other members of the corona-viridae, which are essentially spherical in shape with a diameter of 80 to 160 nm, together with outstanding projections about 25 nm long, which give the viruses the appearance of possessing a halo or corona.

The titre of virus produced from each passage will vary from between about $10^{4.0}$ $TCID_{50}$ and $10^{10.0}TCID_{50}$ per ml. ($TCID_{50}$ means the tissue culture infective dose which produces a cytopathic effect in 50% of the culture cells exposed to virus).

Once the FIP infected cultures exhibit a CPE, the cell culture is harvested and the virus is inactivated by, for example, chemical means such as formaldehyde, acetylethyleneimine, $\beta$-propiolactone, $\beta$-ethyleneimine or other known virus inactivating agents. When formaldehyde is the selected inactivating agent, the virus suspension, obtained after harvesting of the cultures, is incubated in the presence of, for example, 0.08% formaldehyde, until complete inactivation of the virus is achieved; this usually requires more than 72 hours incubation. Following this the formaldehyde is neutralised with for example sodium metabisulphate, and the suspension is dialysed against phosphate buffered saline to remove excess sodium metabisulphate, which otherwise would interfere with subsequent safety tests. Alternatively the virus may be inactivated by physical means such as UV radiation.

Before formulating the inactivated virus into a vaccine, the preparation must first be tested for freedom from live virus, for sterility and potency. To ensure freedom from live virus a sample of the preparation is incubated in feline embryonic cell cultures. Lack of CPE in the cell cultures indicates the absence of live virus. To test for sterility, nutrient and meat broths are inoculated with the suspension. Potency is judged by the antibody titres produced in cats as a result of injecting them with known dilutions of the inactivated virus suspension.

The pharmaceutically acceptable carrier for the vaccine can be a liquid, such as an aqueous solution containing nutrients and stabilizers, e.g. Hank's balanced salt solution or other similar media. The carrier may, in some instances, include a sterile sealed container, such as an ampoule or vial.

According to a second aspect of the invention there is provided a vaccine for developing immunity in animals of the genus *Felix* against disease caused by FIP, which comprises FIP virus propagated, inactivated and formulated, as hereinbefore described, in an effective dosage, or multiples thereof. The effective dosage for vaccination may be from about $10^{4.0}$ to about $10^{8.0}$ $TCID_{50}$/ml preferably $10^{5.0}$ to $10^{7.0}$ $TCID_{50}$/ml. The $TCID_{50}$/ml of the virus preparation is determined prior to inactivation.

Preferably the inactivated FIP virus is combined with an adjuvant for stimulating the immune response of the animal to be vaccinated, and therefore increasing the effectiveness of the vaccine. Suitable adjuvants include aluminium or calcium salts such as the phosphate or silicate; aluminium hydroxide gel is preferably used. An alternative type of adjuvant is that of the oil-water-lanolin type such as that described by Freund or mineral oil mixtures such as Bayol F (Esso Ltd.) and Falba (Pfaltz and Bauer Inc. New York). Vaccines may be prepared by homogenising the selected oily adjuvant with an equal volume of the inactivated virus preparation.

Prior to formulating the vaccine the virus may be concentrated by any of several means including for example diafiltration with a protein precipitant such as organic solvents. Good results can be achieved with water miscible lower aliphatic alcohols and ketones, particularly methanol and acetone.

After formulation the vaccine is conveniently incorporated into a sterile container, which is then sealed and stored at 2 to 8°C until the vaccine is required for use. Alternatively the virus suspension may be frozen in ampoules or vials, and stored in liquid nitrogen. A further alternative is to add stabilizers, such as sorbitol and/or protein hydrolystate, e.g. Sol-u-pro, to the virus suspension to enable the preparation to be freeze dried.

The vaccine of the present invention is administered to animals of the genus *Felix* desirably by intraperitoneal, subcutaneous or intramuscular injection or via the oral, nasal or intraocular route. The dose may be administered as a single unit, or as a multiplicity of sub-doses over a period of time. The preferred schedule is to administer two doses of 2 ml containing the effective dosage of FIP virus, which are administered at not less than 21 days apart.

In addition to containing the attenuated FIP virus, the vaccine of the present invention may also contain other vaccines comprising antigenic material derived from other microorganisms that cause disease in members of the genus *Felix*, such as parvoviruses for example feline panleucopenia (feline infectious enteritis), feline calicivirus, feline rabies, feline retrovirus, e.g. feline leukaemia virus, and feline herpes viruses.

A particularly preferred combination, presented as a vaccine pack, comprises a wet preparation of inactivated FIP virus in a sterile vessel together with a vessel containing a freeze dried preparation of one or more feline vaccines suitably adapted to be combined before usage. Alternatively the inactivated FIP virus may be freeze-dried in combination with the other vaccine viruses and packaged with a vessel containing sterile water for use in reconstituting the freeze-dried vaccine prior to administration. The vessels are packaged in a box or container together with instructions for use, which include the instruction to reconstitute the freeze-dried vaccine with the wet preparation or sterile water prior to administration.

In a further aspect of the present invention there is provided a method for developing immunity in animals against FIP which comprises the administration to the animal of an effective dosage of a vaccine as hereinbefore defined.

The following Examples illustrate the invention but do not limit it in any way.

### Example 1
Preparation of Inactivated Virus
  a) *Propagation*
A 6 month old cat suffering from FIP, as demonstrated by pyrexia and loss of condition, was bled from the jugular vein. The blood was left for 20 minutes to allow it to clot. The serum/plasma was removed from the clot, the clot was then triturated in a tissue disintegrater, i.e. a Stomacher (COLWORTH) in phosphate buffered saline for 60 seconds. The suspension was then centrifugred at 1108 g after which the supernatant was decanted and used to inoculate confluent monolayers of diploid feline embryonic lung cells in serum-free 199 Medium and incubated at 37°C for approximately 7 days. After incubation the cells were harvested by freezing and thawing. An aliquot of the medium and disrupted infected cells was then inoculated on to fresh monolayer cultures of diploid feline embryonic lung cells in 199 Medium, and incubated at 37°C.

After serially passaging the virus in a similar manner six times, small areas of characteristic CPE started to appear, that is the infected cells produced irregular shapes and eventually rounded up when viewed as wet preparations under the microscope.

From the fifth passage onwards the CPE was very evident and rapidly destroyed the cell sheet, that is within 48 to 72 hours.

  b) *Inactivation*
After the sixth passage the infected cells were harvested by freezing and thawing and gave a virus titre of $10^{9.0}$ TCID$_{50}$/ml. An aliquot (100 ml) of the harvest was mixed with 40% formaldehyde (0.4 ml) and stood on a magnetic stirrer at 4°C for 96 hours, after which time the virus was totally inactivated. Sodium metabisulphate (2 ml) at 1:16 of standard 32% was added to the virus preparation to neutralize the formaldehyde. In order to remove the sodium metabisulphate for the purposes of carrying out the safety test, the virus preparation was dialysed overnight in Sorensons phosphate buffered saline.

  c) *Control Standard*
  (i) Sterility Test
Aliquots (5 ml) of the inactivated virus preparation were added to 300 ml of nutrient broth and meat broth respectively and incubated at 32°C for 7 days and then at 18 to 20°C for a further 7 days. No growth was observed in either medium showing that the preparation was free from contamination.

  (ii) Safety Test
Diploid Feline embryonic lung cells were inoculated with the inactivated virus preparation and incubated at 37°C. After 7 days the cells were versenized and re-seeded and incubated at 37°C for a further 7 days. No CPE was observable at 72 hours or 96 hours indicating that there was no virus replication.

### Example 2
Peritoneal fluid was removed aseptically from a 3 to 4 month old cat which had died from FIP, by pipette and inoculated on to confluent monolayers of diploid feline whole embryo cells in serum free 199 Medium and incubated at 37°C for 7 days.

The virus was then harvested, passaged and inactivated as described in Example 1.

### Example 3
The liver was removed from an 11 month old cat, which had died from FIP. The tisue was triturated in a tissue disintegrater, Stomacher 80 (COLWORTH) with phosphate buffered saline for 60 seconds. The suspension was then centrifuged at 1108 g after which the supernatant was decanted and inoculated onto confluent monolayers of heteroploid feline embryonic lung cells in serum free 199 Medium and incubated at 37°C for 7 days. (Heteroploid cells are cells which have been passaged more than 70 times from primary culture and have, in consequence, become an established cell line.)

The virus was then harvested, passaged and inactivated as described in Example 1.

### Example 4
Preparation of FIP Vaccine
Inactivated viral material having a titre of $10^{9.0}$ TCID$_{50}$/ml prior to inactivation was prepared and tested as described in Example 1. Equal volumes of the virus preparation, alhydrogel and phosphate buffered saline were mixed together and agitated on a magnetic stirrer for 1 hour at top speed.

### Example 5
A vaccine, as prepared in Example 4 was administered to 12 week old cats, which had previously been shown to be free of FIP antibody, by intraperitoneal injection as a dose of 2 ml. After 21 days a further 2 ml dose of vaccine was administered intraperitoneally; at this time the cats were also bled and the sera examined for the presence of neutralising FIP antibody. The cats were similarly bled 21 days after the second injection. Significant levels of neutralising antibody were found to be present as was demonstrated below:—

Pre-vaccination antibody titre = less than 10

Antibody titre 21 days after 1st injection = 40

Antibody titre 21 days after 2nd injection = 80.

### Example 6

A vaccine pack for immunising members of the genus *felix* against FIP and feline infectious enteritis (FIE) is composed of a vial containing a freeze dried preparation of live attenuated FIE vaccine and a second vial containing an inactivated FIP virus suspension as prepared in Example 4, together with instructions to reconstitute the freeze-dried vaccine with the wet preparation prior to administration.

### Example 7

A vaccine pack for immunising members of the genus *felix* against FIP, feline infectious enteritis (FIE) and feline viral rhinotracheitis is composed of a vial containing a freeze-dried preparation of live attenuated FIE vaccine and live modified feline viral rhinotracheitis and a second viral containing an inactivated FIP virus suspension, as prepared in Example 4, together with instructions to reconstitute the freeze-dried vaccine with the wet preparation prior to administration.

### Claims

1. A vaccine for developing immunity in animals to feline infectious peritonitis comprising an immunologically effective dosage of inactivated feline infectious peritonitis virus in association with a pharmaceutically acceptable carrier.

2. A vaccine as claimed in claim 1 characterised in that the effective dosage is from $10^{4.0}$ to $10^{8.0}$ TCID$_{50}$ per ml, where TCID$_{50}$ means the tissue culture infective dose which produces a cytopathic effect in 50% of the culture cells exposed to virus.

3. A method for preparing an inactivated feline infectious peritonitis vaccine as claimed in claim 1 comprising culturing virulent virus in feline embryonic cell cultures, inactivating the virus so produced and presenting the inactivated virus in association with a pharmaceutically acceptable carrier characterised in that virulent infectious peritonitis virus is serially passaged through about five passages until the infected cultures exhibit a cytopathic effect, that is the cells produce irregular shapes when examined microscopically as wet preparations.

4. A method as claimed in claim 3 further characterised in that the embryonic cell cultures comprise diploid feline embryonic lung cells.

5. A method as claimed in claim 3 further characterised in that the feline embryonic cell cultures comprise heteroploid feline embryonic lung cells.

6. A method as claimed in any one of claims 3 to 5 further characterised in that the virus is inactivated by chemical means.

7. A method as claimed in any one of claims 3 to 5 further characterised in that the virus is inactivated by physical means.

### Patentansprüche

1. Impfstoff zur Erzeugung von Immunität in Tieren gegen infektiöse Katzen-Peritonitis, enthaltend eine immunologisch wirksame Dosis des inaktivierten, infektiösen Katzen-Peritonitis-Virus in Kombination mit einem pharmazeutisch annehmbaren Träger.

2. Impfstoff nach Anspruch 1, dadurch gekennzeichnet, daß die wirksame Dosis $10^{4.0}$ bis $10^{8.0}$ TCID$_{50}$ pro ml beträgt, wobei TCID$_{50}$ für die infektiöse Gewerbekulturdosis steht, die bei 50 % der dem Virus ausgesetzten Zellen der Kultur einen cytopathischen Effekt verursacht.

3. Verfahren zur Herstellung des inaktivierten, infektiösen Katzen-Peritonitis-Impfstoffs nach Anspruch 1, durch Züchten des virulenten Virus in embryonalen Katzenzellkulturen, Inaktivieren des in dieser Weise erzeugten Virus und Kombinieren des inaktivierten Virus mit einem pharmazeutisch annehmbaren Träger, dadurch gekennzeichnet, daß man das virulente, infektiöse Peritonitis-Virus nacheinander durch etwa fünf Durchgänge führt, bis die infizierten Kulturen einen cytopathischen Effekt zeigen, d. h. die Zellen unregelmäßige Formen aufweisen, wenn sie in Form von Naßpräparaten mikroskopisch untersucht werden.

4. Verfahren nach Anspruch 3, dadurch gekennzeichnet, daß man also embryonale Zell-kulturen diploide embryonale, Katzenlungen-zellen verwendet.

5. Verfahren nach Anspruch 3, dadurch gekennzeichnet, daß man als embryonale Katzenzellkulturen heteroploide, embryonale Katzenlungenzellen verwendet.

6. Verfahren nach einem der Ansprüche 3 bis 5, dadurch gekennzeichnet, daß man das Virus chemisch inaktiviert.

7. Verfahren nach einem der Ansprüche 3 bis 5, dadurch gekennzeichnet, daß man das Virus physikalisch inaktiviert.

### Revendications

1. Vaccin pour conférer l'immunité à des animaux à l'égard de la péritonite infectieuse des félins, qui comprend une dose immunologique- ment efficace de virus inactivé de la péritonite infectieuse des félins en association avec un excipient pharmaceutiquement acceptable.

2. Vaccin suivant la revendication 1, caractérisé en ce que la dose efficace est de $10^{4,0}$ à $10^{8,0}$ $DI_{50}CT/ml$, où $DI_{50}CT$ signifie la dose infectieuse pour la culture tissulaire qui produit un effet cytopathogène chez 50% des cellules en culture exposées au virus.

3. Procédé de préparation d'un vaccin inactivé contre la péritonite infectieuse des félins suivant la revendication 1 qui comprend la mise en culture du virus virulent dans des cultures de cellules d'embryon de félin, l'inactivation du virus ainsi obtenu et la présentation du virus inactivé en association avec un excipient pharmaceutiquement acceptable, caractérisé en ce que le virus virulent de la péritonite infectieuse est soumis à des passages en série pour environ cinq passages jusqu'à ce que les cultures infectées manifestent un effet cytopathogène, c'est-à-dire que les cellules acquièrent des formes irrégulières lorsqu'elles sont examinées au microscope à l'état de préparation humide.

4. Procédé suivant la revendication 3, caractérisé en ce que les cultures de cellules d'embryon comprennent des cellules de poumon d'embryon de félin diploïdes.

5. Procédé suivant la revendication 3, caractérisé en ce que les cultures de cellules d'embryon de félin comprennent des cellules de poumon d'embryon de félin hétéroploïdes.

6. Procédé suivant l'une quelconque des revendications 3 à 5, caractérisé en ce que le virus est inactivé par des moyens chimiques.

7. Procédé suivant l'une quelconque des revendications 3 à 5, caractérisé en ce que le virus est inactivé par des moyens physiques.